# EUROPÄISCHE PATENTSCHRIFT

(11) **EP 2 689 800 B1**
(45) Veröffentlichungstag und Bekanntmachung des Hinweises auf die Patenterteilung: **23.09.2015**
(21) Anmeldenummer: 12177675.1
(22) Anmeldetag: 24.07.2012
(51) Int. Cl.: A61N 5/06

(54) **Geräteständer für eine Strahlentherapievorrichtung, Strahlentherapievorrichtung und -system**
Support d'appareil pour un dispositif de thérapie par rayonnement, dispositif et système de thérapie par rayonnement
Device stand for a radiotherapy device, radiotherapy device and system

(43) Veröffentlichungstag der Anmeldung: 29.01.2014
(73) Patentinhaber: Dr. Hönle Medizintechnik GmbH, 86916 Kaufering (DE)
(72) Erfinder: Kleinhans, Petra, 86899 Landsberg am Lech (DE)
(74) Vertreter: Hofstetter, Schurack & Partner

(56) Entgegenhaltungen:
- DE-T2- 69 023 237
- DE-U1- 8 716 290
- DE-U1- 20 013 335
- DE-U1-202012 001 280

## Beschreibung

Die vorliegende Erfindung betrifft einen Geräteständer für eine Strahlentherapievorrichtung, insbesondere für die UV-Bestrahlung und UV-Therapie, mit mindestens einer Tragsäule umfassend mindestens einen Feststellmechanismus zur lösbaren Befestigung von mindestens einem Bestrahlungsgerät, wobei die Tragsäule an einem ungefähr U-, sichel- oder halbkreisförmigen sowie verfahrbaren Fußgestell abgestützt ist. Die Erfindung betrifft weiterhin eine Strahlentherapievorrichtung sowie ein Strahlentherapiesystem.

Geräteständer der eingangs genannten Art sind aus der DE 20 2012 001280 U1 bekannt. Nachteilig an diesen Geräteständern ist jedoch, dass diese nur sehr eingeschränkt und gerätespezifisch verwendet werden können. Des Weiteren sind Bestrahlungsgeräte, insbesondere UV-Bestrahlungsgeräte für die Ganzkörperbestrahlung bzw. -therapie bekannt. So beschreibt die DE 78 26 093 U1 ein Bestrahlungsgerät mit mehreren röhrenförmigen, ultraviolette Strahlung abgebenden Niederdruckstrahlern. Dabei kann das Bestrahlungsgerät aus zwei Kammerhälften bestehen, die zusammen eine Kammer als Bestrahlungsraum ausbilden und über ein zentrales Scharnier gelenkig miteinander verbunden sind. Nachteilig an diesem bekannten Bestrahlungsgerät ist jedoch, dass auch dieses nur sehr eingeschränkte Anwendungsmöglichkeiten für die Strahlentherapie, nämlich ausschließlich für die

UV-Ganzkörperbestrahlung bereitstellt. Zudem ist das bekannte Bestrahlungssystem insgesamt sperrig ausgebildet, kann nur im Ganzen transportiert werden und benötigt einen entsprechend großen Stellraum.

Es ist daher eine Aufgabe der vorliegenden Erfindung, einen Geräteständer der eingangs genannten Art bereitzustellen, welcher erweiterte Anwendungsmöglichkeiten, insbesondere auch in Kombination mit weiteren Geräteständern gleicher oder ähnlicher Bauart gewährleistet.

Es ist weiterhin Aufgabe der Erfindung eine Strahlentherapievorrichtung sowie ein Strahlentherapiesystem der eingangs genannten Art bereitzustellen, welche erweiterte Anwendungsmöglichkeiten, insbesondere auch in Kombination mit weiteren Strahlentherapievorrichtungen gleicher oder ähnlicher Bauart gewährleistet.

Gelöst werden diese Aufgaben durch einen Geräteständer mit den Merkmalen des Patentanspruchs 1 sowie einer Strahlentherapievorrichtung mit den Merkmalen des Patentanspruchs 12 sowie einem Strahlentherapiesystem mit den Merkmalen des Patentanspruchs 14. Vorteilhafte Ausgestaltungen der Erfindung sind in den jeweiligen Unteransprüchen angegeben.

Ein erfindungsgemäßer Geräteständer für eine Strahlentherapievorrichtung, insbesondere für die UV-Bestrahlung und UV-Therapie, umfasst mindestens eine Tragsäule mit mindestens einem Feststellmechanismus zur Befestigung von mindestens einem Bestrahlungsgerät, wobei die Tragsäule an einem ungefähr U-, sichel- oder halbkreisförmigen, verfahrbaren Fußgestell abgestützt ist. Dabei weist das Fußgestell mindestens im Bereich eines von der Tragsäule wegweisenden Endes mindestens ein hakenförmiges Verbindungselement auf. Durch die erfindungsgemäße Ausbildung des Verbindungselementes ist es möglich, zwei oder mehrere Geräteständer miteinander zu verbinden bzw. zu koppeln. Dies wiederum bewirkt, dass mehrere Strahlentherapievorrichtungen über die jeweiligen erfindungsgemäßen Geräteständer definiert zueinander angeordnet werden können. So können zum Beispiel zwei Strahlentherapievorrichtungen im ungefähr rechten Winkel zueinander angeordnet werden. Dies bewirkt, dass eine größere Körperoberfläche eines sich zwischen den beiden zueinander angeordneten Strahlentherapievorrichtungen befindlichen Benutzers gezielt bestrahlt werden kann. Es ist aber auch möglich, dass zum Beispiel durch die Verbindung dreier Geräteständer mit entsprechend angeordneten, in ihrer Längserstreckung vertikal angeordneten Strahlentherapievorrichtungen einen kabinenartigen Raum umschließen, in dem der Benutzer sich für den Zeitraum der Strahlenbehandlung aufhalten kann. Auch können zum Beispiel vier miteinander verbundene Geräteständer mit entsprechenden Strahlentherapievorrichtungen einen zumindest zeitweise relativ geschlossenen Raum für die Strahlenbehandlung ausbilden. Damit ist der erfindungsgemäße Geräteständer vielfach verwendbar und bietet entsprechend erweiterte Anwendungsmöglichkeiten. Neben der Verwendung des erfindungsgemäßen Geräteständers als Einzelgerät ergibt sich auch durch die definierte Verbindung mehrerer erfindungsgemäßer Geräteständer eine Verwendung als Mehrgerätesystem. Dadurch dass der Geräteständer verfahrbar ausgebildet ist, können die jeweiligen Einzelmodule der Strahlentherapievorrichtung bestehend aus dem erfindungsgemäßen Geräteständer und dem mindestens einen daran angeordneten Bestrahlungsgerät leicht und ohne weiteres verschoben und zueinander angeordnet werden. Unterschiedlichste Behandlungsformen können dadurch abgedeckt werden, da die erfindungsgemäßen Geräteständer mit daran entsprechend befestigten Bestrahlungsgeräten über die genannten Verbindungselemente in einem definierten Abstand und Winkel zueinander angeordnet werden können. Somit können gezielt vordefinierte Hautpartien eines Benutzers bestrahlt werden.

In weiteren vorteilhaften Ausgestaltungen des erfindungsgemäßen Geräteständers ist das Verbindungselement als separates Element ausgebildet und am oder im Fußgestell lösbar oder nicht-lösbar befestigt. Dabei kann zum Beispiel das Fußgestell mindestens in dem Bereich eines des von der Tragsäule wegweisenden Endes hohlförmig zur Aufnahme des Verbindungselementes ausgebildet sein. Des Weiteren besteht die Möglichkeit, dass dabei das hakenförmige Verbindungselement derart in dem Ende des Fußgestells angeordnet ist, dass ein nicht innerhalb des Fußgestells angeordnetes Ende des Verbindungselements in einer von dem Fußgestell wegzeigenden Richtung verläuft. Entsprechendes gilt auch für Verbindungselemente, die am Fußgestell lösbar oder nicht-lösbar befestigt sind sowie für einstückig mit dem jeweiligen Ende des Fußgestells ausgebildete Verbindungselemente. Durch die genannten Ausführungsformen ist eine Vielzahl von Befestigungsmöglichkeiten des Verbindungselements an dem Fußgestell möglich. Diese können je nach Anforderung auf die gerätespezifischen Besonderheiten der an dem Geräteständer befestigten Bestrahlungsgeräte ausgebildet sein. Des weiteren ist durch die Ausgestaltung bzw. Anordnung der Verbindungselemente eine einfache Verbindung zweier oder mehrerer erfindungsgemäßer Geräteständer möglich, da die nach außen weisenden und miteinander zu verbindenden Enden der jeweiligen Verbindungselemente einfach, sicher und schnell durch entsprechende Feststell- und Befestigungsmechanismen verbunden werden können. Das Verbindungselement des erfindungsgemäßen Geräteständers kann dabei mindestens einen Feststell- und Befestigungsmechanismus zum Verbinden eines Endes des Verbindungselementes eines ersten erfindungsgemäßen Geräteständers mit einem entsprechenden Ende eines Verbindungselementes eines zweiten erfindungsgemäßen Geräteständers aufweisen.

In weiteren vorteilhaften Ausgestaltungen des erfindungsgemäßen Geräteständers ist das Verbindungselement innerhalb des von der Tragsäule wegweisenden Endes längenverstellbar angeordnet. Hierzu kann das von der Tragsäule wegweisende Ende des Fußgestells mindestens zwei voneinander beabstandete Öffnungen zur Aufnahme eines Feststellbolzens aufweisen, wobei der Feststellbolzen in eine entsprechende Öffnung in dem Verbindungselement einbringbar ist. Die Öffnungen in dem wegweisenden Ende des Fußgestells können dabei auf beiden Seiten des Endes oder nur auf einer Seite des entsprechenden Endes des Fußgestells ausgebildet sein. Durch die Längenverstellbarkeit des Verbindungselementes relativ zum Geräteständer kann der Abstand miteinander verbundener Geräteständer individuell eingestellt werden. Dies ist insbesondere dann von Vorteil, wenn der Abstand der an den Geräteständern befestigten Bestrahlungsgeräte zueinander oder auch zu einem Benutzer festgelegt werden muss.

In weiteren vorteilhaften Ausgestaltungen des erfindungsgemäßen Geräteständers weisen die Tragsäule und/oder das Fußgestell einen quadratischen, rechteckigen oder kreisförmigen Querschnitt auf. Aber auch andere Querschnittsformen sind denkbar. Des Weiteren ist es möglich, dass die Tragsäule höhenverstellbar ausgebildet ist. Hierzu kann zum Beispiel die Tragsäule aus mindestens zwei ineinander steckbaren und verschiebbaren, rohrförmigen Elementen bestehen. Es ergibt sich dadurch ein teleskopartiger Aufbau der Tragsäule. Vorteilhafterweise können durch die Höhenverstellbarkeit der Tragsäule die daran befestigten Bestrahlungsgeräte exakt auf die jeweiligen Anforderungen ausgerichtet werden. Es ist aber auch möglich, dass die Tragsäule entlang ihrer Längserstreckung eine Vielzahl von Bohrungen oder anders gearteten Feststellmechanismen aufweist, sodass das oder die Bestrahlungsgeräte in unterschiedlichen Höhen angeordnet und an der Tragsäule befestigt werden können. Für den Fall, dass die Tragsäule Bohrungen in unterschiedlicher Höhe aufweist, kann das oder die Bestrahlungsgeräte mittels entsprechender Schrauben oder Bolzen an der Tragsäule befestigt werden.

In einer weiteren vorteilhaften Ausgestaltung des erfindungsgemäßen Geräteständers umfasst das Fußgestell einen Querträger und zwei ungefähr senkrecht von den Enden des Querträgers abzweigende Seitenarme. Durch die ungefähr U-förmige Ausgestaltung des Fußgestells ist einerseits ein sicherer Stand des Geräteständers mit dem oder den daran befestigten Bestrahlungsgeräten gewährleistet. Zudem entsteht durch die U-förmige Ausgestaltung des Fußgestells ein Zwischenraum, der durch den Benutzer des an dem Geräteständer befestigten Bestrahlungsgerätes genutzt werden kann.

In einer weiteren vorteilhaften Ausgestaltung des erfindungsgemäßen Geräteständers ermöglicht der Feststellmechanismus zur lösbaren Befestigung des mindestens einen Bestrahlungsgerätes ein Schwenken des Bestrahlungsgerätes.

Dadurch können wiederum gezielt vorbestimmte Hautpartien bestrahlt werden. Des Weiteren ermöglicht zum Beispiel eine Schwenkbarkeit um 90° Anwendungen im Stehen, Liegen und Sitzen. Auch lokale Therapien, zum Beispiel im Rücken- oder Nackenbereich können problemlos durchgeführt werden.

Die vorliegende Erfindung betrifft weiterhin eine Strahlentherapievorrichtung, insbesondere für die UV-Bestrahlung und UV-Therapie, welche mindestens ein Bestrahlungsgerät, welches an einem Geräteständer wie im Vorhergehenden beschrieben befestigt ist, insbesondere lösbar befestigt ist. Die Strahlentherapievorrichtung kann durch den erfindungsgemäßen Geräteständer entweder als Einzelmodul oder als Mehrfachmodul verwendet werden. Durch ein Verbinden zweier oder mehrerer Strahlentherapievorrichtungen mittels der im Vorhergehenden beschriebenen Geräteständer ist es möglich, einen definierten Bestrahlungsraum auszubilden. Der Bestrahlungsraum kann dabei geschlossen oder nach einer Seite hin offen ausgebildet sein. Vorteilhafterweise ergeben sich dadurch eine Vielzahl an Verwendungsmöglichkeiten der erfindungsgemäßen Strahlentherapievorrichtung, die individuell an die jeweilige Behandlungsmethode angepasst werden kann.

In einer vorteilhaften Ausgestaltung der Strahlentherapievorrichtung ist das Bestrahlungsgerät in der Draufsicht gekrümmt oder gebogen ausgebildet. Diese Ausgestaltung hat sich als besonders ergonomisch erwiesen.

Die weiteren im Vorhergehenden beschriebenen Vorteile und Anwendungsmöglichkeiten des erfindungsgemäßen Geräteständers treffen entsprechend auch auf die erfindungsgemäße Strahlentherapievorrichtung zu.

Die Erfindung umfasst weiterhin ein Strahlentherapiesystem bestehend aus mindest zwei über ihre Geräteständer lösbar miteinander verbundene Strahlentherapievorrichtungen wie sie im Vorhergehenden beschrieben worden sind. Das erfindungsgemäße Strahlentherapiesystem ist flexibel einsetzbar und gewährleistet eine Vielzahl von Anwendungsmöglichkeiten, insbesondere durch die Kombination mit Strahlentherapievorrichtungen gleicher oder ähnlicher Bauart. Durch die im Vorhergehenden beschriebenen erfindungsgemäßen Geräteständer ist es möglich, die Strahlentherapievorrichtungen definiert zueinander anzuordnen. So können die Strahlentherapievorrichtungen bezüglich ihrer Abstände und Winkel zueinander fest miteinander verbunden werden. Des Weiteren ist es möglich, mindestens drei Strahlentherapievorrichtungen miteinander zu koppeln, derart, dass sie einen kabinenartigen Raum für den Aufenthalt eines Benutzers des Strahlentherapiesystems umschließen. Durch die Ausbildung eines kabinenartigen Raums kann auf festinstallierte Bestrahlungssysteme, insbesondere Ganzkörperbestrahlungssysteme verzichtet werden. Insbesondere ergibt sich dadurch eine Raumersparnis, da das erfindungsgemäße Strahlentherapiesystem einfach durch Lösen der einzelnen Strahlentherapievorrichtungen voneinander abgebaut und platzsparend gelagert werden kann.

Weitere Merkmale der Erfindung ergeben sich aus den Ansprüchen, dem Ausführungsbeispiel sowie anhand der Zeichnungen. Die vorstehend in der Beschreibung genannten Merkmale und Merkmalskombinationen sowie die nachfolgend in dem Ausführungsbeispiel genannten Merkmale und Merkmalskombinationen sind nicht nur in der jeweils angegebenen Kombination, sondern auch in anderen Kombinationen verwendbar, ohne den Rahmen der Erfindung zu verlassen. Dabei zeigt:
- Figur 1: ein schematisch dargestellter, erfindungsgemäßer Geräteständer gemäß einer ersten Ausführungsform;
- Figur 2: ein schematisch dargestellter, erfindungsgemäßer Geräteständer gemäß einer zweiten Ausführungsform;
- Figur 3: eine schematische Detaildarstellung eines erfindungsgemäßen Strahlentherapiesystems;
- Figur 4: eine schematische Darstellung einer erfindungsgemäßen Strahlentherapievorrichtung;
- Figur 5: eine schematische Darstellung zweier miteinander verbundener, erfindungsgemäßer Geräteständer gemäß Figur 2; und
- Figur 6: eine schematische Gesamtdarstellung des erfindungsgemäßen Strahlentherapiesystems gemäß Figur 3.

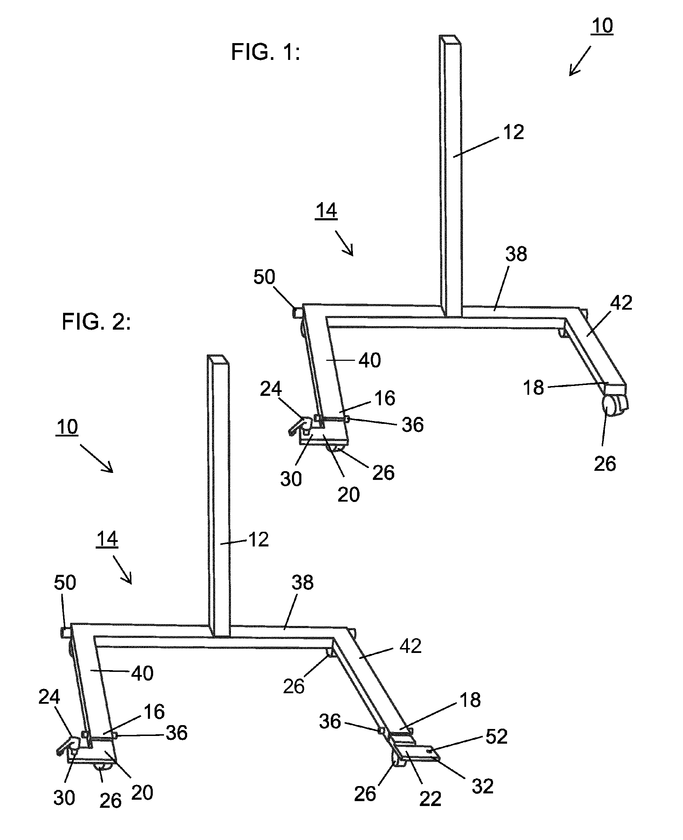

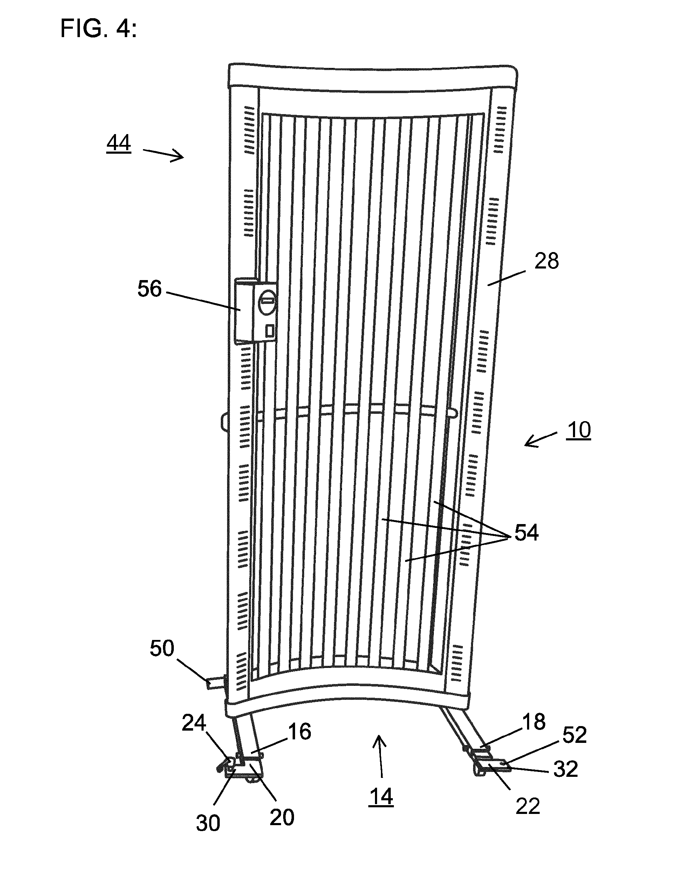

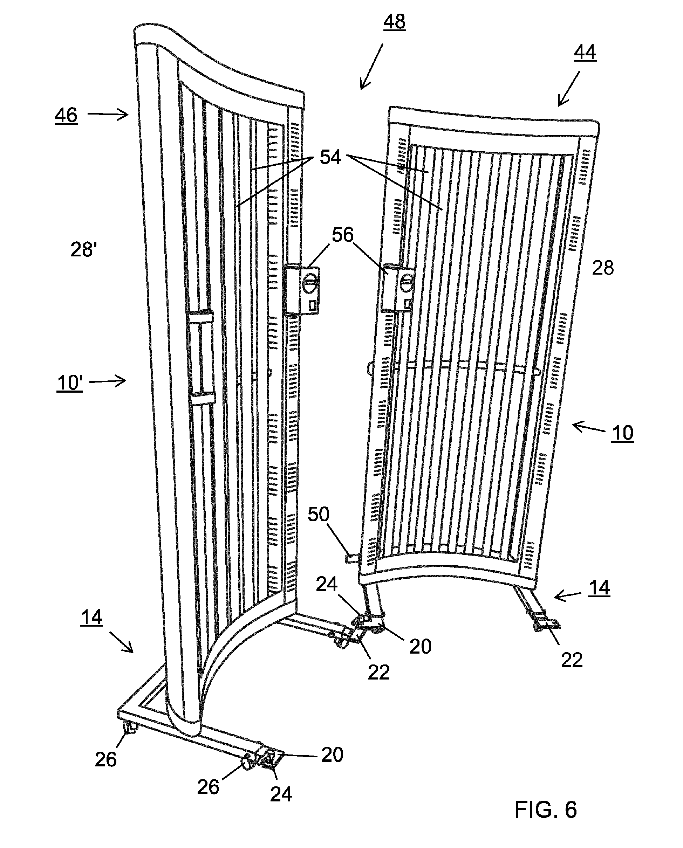

Die Figur 1 zeigt einen schematisch dargestellten Geräteständer 10 gemäß einer ersten Ausführungsform. Es handelt sich dabei um einen Geräteständer 10 für eine Strahlentherapievorrichtung (vergleiche insbesondere Figuren 4 und 6). Der Geräteständer 10 umfasst dabei eine Tragsäule 12 zur lösbaren Befestigung von mindestens einem Bestrahlungsgerät 28, 28' (vergleiche Figur 6). Hierfür kann die Tragsäule 12 mindestens einen Feststellmechanismus (nicht dargestellt) aufweisen. Der Feststellmechanismus kann ich einfachster Weise aus entsprechenden Bohrungen in der Tragsäule 12, in die entsprechende Feststellschrauben oder -bolzen eingreifen, bestehen. Es ist aber auch möglich, dass der Feststellmechanismus zur lösbaren Befestigung des mindestens einen Bestrahlungsgerätes 28, 28' ein Schwenken des Bestrahlungsgerätes 28, 28' ermöglicht.

Des Weiteren erkennt man, dass die Tragsäule 12 an einen ungefähr U-förmigen und verfahrbaren Fußgestell 14 abgestützt ist. Das Fußgestell 14 kann aber auch sichel- oder halbkreisförmig ausgebildet sein. Auch andere vergleichbare Formen sind denkbar. In dem dargestellten Ausführungsbeispiel umfasst das Fußgestell 14 einen Querträger 38 und zwei ungefähr senkrecht von den Enden des Querträgers 38 abzweigende Seitenarme 40, 42. Man erkennt, dass die Tragsäule 12 im Querschnitt quadratisch und die Elemente des Fußgestells 14 im Querschnitt rechteckig ausgebildet sind. Es ist aber auch möglich im Querschnitt kreisförmige oder ovale oder auch weitere vieleckförmige Elemente zur Ausgestaltung der Tragsäule 12 und des Fußgestells 14 zu verwenden. Insbesondere bestehen die Tragsäule 12 und das Fußgestell 14 aus Metall, einer Metalllegierung oder Stahl. Auch andere geeignete Materialien sind denkbar.

Der Geräteständer 10 ist verfahrbar ausgebildet und weist hierfür Rollen 26 auf, die an dem Fußgestell 14 entsprechend angeordnet sind. Die Rollen 26 können Feststellbremsen aufweisen.

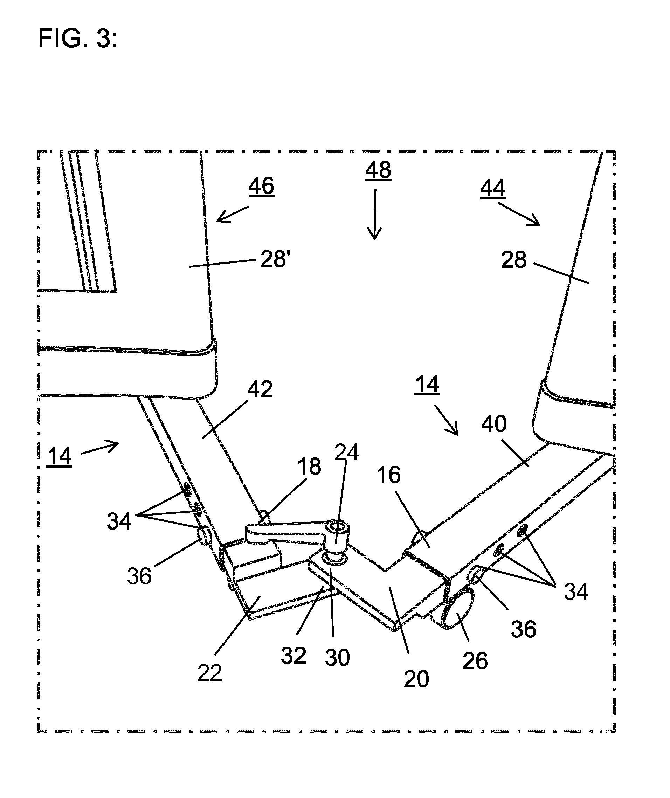

Des Weiteren erkennt man, dass der Geräteständer 10 gemäß dem ersten Ausführungsbeispiel im Bereich eines von der Tragsäule 14 wegweisenden Endes 16 des Fußgestells 14 ein hakenförmiges Verbindungselement 20 aufweist. In dem dargestellten Ausführungsbeispiel ist das Verbindungselement 20 als separates Element ausgebildet und im Fußgestell 14 lösbar befestigt. Das Verbindungselement 20 ist dabei innerhalb des von der Tragsäule 14 wegweisenden Endes 16 längenverstellbar befestigt. Hierfür sind in dem Ende 16 des Fußgestells 14 mehrere voneinander beabstandete Öffnungen 34 zur Aufnahme eines Feststellbolzens 36 ausgebildet (vergleiche auch Figur 3). Der Feststellbolzen 36 ist dabei in eine entsprechende Öffnung in dem Verbindungselement 20 einbringbar.

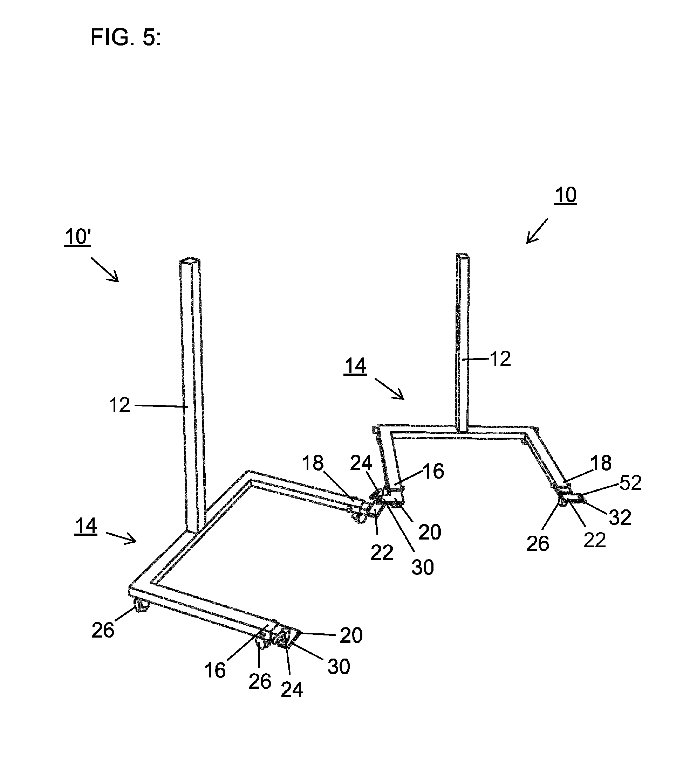

Schließlich weist das Fußgestell 14 im Bereich der Enden des Querträgers 38 angeordnete Abstandselemente 50 auf, welche einen Mindestabstand zwischen einzelnen über die hakenförmige Verbindungselemente 20, 22 zu verbindenden Geräteständer 10, 10' (vergleiche auch Figur 5) festlegen. Für die Verbindung der Geräteständer 10, 10' weisen die Verbindungselemente 20, 22 einen Feststell- und Befestigungsmechanismus 24 zur Verbindung ihrer jeweiligen Enden 30, 32 auf (vergleiche Figur 3). In dem dargestellten ersten Ausführungsbeispiel des Geräteständers 10 weist das Fußgestell 14 nur an einem Ende 16 ein Verbindungselement 20 auf. Das andere Ende 18 des Fußgestells 14 ist ohne Verbindungselement ausgebildet.

Figur 2 zeigt eine schematische Darstellung eines Geräteständers 10 gemäß einer zweiten Ausführungsform. Man erkennt, dass im Gegensatz zu der in Figur 1 dargestellten Ausführungsform diese nunmehr zwei Verbindungselemente 20, 22 aufweist. Das Verbindungselement 20 ist wiederum im Bereich des von der Tragsäule 12 wegweisenden Endes 16 angeordnet. Das zweite Verbindungselement 22 ist dagegen im Bereich des von der Tragsäule 14 wegweisenden Endes 18 angeordnet. Die Verbindungselemente 20, 22 sind wiederum als separate Elemente ausgebildet und im Fußgestell 14 befestigt. Hierfür ist das Fußgestell 14 mindestens im Bereich der von der Tragsäule 12 wegweisenden Enden 16, 18 hohlförmig ausgebildet. Man erkennt, dass die hakenförmigen Verbindungselemente 20, 22 derart in den Enden 16, 18 des Fußgestells 14 angeordnet sind, dass sich die nicht innerhalb des Fußgestells 14 angeordneten Enden 30, 32 der hakenförmigen Verbindungselemente 20, 22 in einer vom Fußgestell 14 wegzeigenden Richtung verlaufen. Diese nach außen, d.h. vom Geräteständer 10 wegweisende Ausgestaltung der Enden 30, 32 der Verbindungselemente 20, 22 ermöglichen eine einfache Verbindung mehrerer Geräteständer 10, 10' über die entsprechenden Verbindungselemente 20, 22, wobei sich die jeweiligen Enden 30, 32 benachbarter Verbindungselemente 20, 22 gegenüberliegen. Zur Verbindung der Verbindungselemente 20, 22 ist wiederum ein Feststell- und Befestigungsmechanismus 24 vorgesehen. In dem dargestellten Ausführungsbeispiel greift ein im Ende 30 des Verbindungselementes 20 ausgebildeter Schraubbolzen in eine entsprechende Öffnung 52 eines Endes 32 eines Verbindungselementes 22 eines zweiten Geräteständers 10' (vgl. Figur 3).

Bezüglich der weiteren Merkmale des Geräteständers 10 gemäß der zweiten Ausführungsform wird auf die Merkmalsbeschreibung der ersten Ausführungsform des Geräteständers 10 gemäß Figur 1 verwiesen.

Figur 3 zeigt eine schematische Detaildarstellung eines Strahlentherapiesystems 48. Das Strahlentherapiesystem 48 besteht dabei in dem dargestellten Ausführungsbeispiel aus zwei Strahlentherapievorrichtungen 44, 46, die über die Geräteständer 10, 10' miteinander gekoppelt sind. An den Geräteständern 10, 10' ist dabei jeweils ein Bestrahlungsgerät 28, 28' angeordnet. Die Verbindung der beiden Geräteständer 10, 10' bzw. der entsprechenden Strahlentherapievorrichtungen 44, 46 erfolgt wiederum über die Verbindungselemente 20, 22. Diese sind über den Feststell- und Befestigungsmechanismus 24 miteinander verbunden. In dem dargestellten Ausführungsbeispiel sind die Verbindungselemente 20, 22 ungefähr L-förmig ausgebildet. Es ist aber auch möglich, dass zur Ausgestaltung der Hakenform auch S- oder C-förmige Verbindungselemente Verwendung finden. Auch andere hakenähnliche Formen sind denkbar. Des Weiteren wird deutlich, dass die Verbindungselemente 20, 22 der beiden Geräteständer 10, 10' unterschiedlich ausgebildet sind, derart, dass ihre jeweiligen Enden 30, 32 übereinander zu liegen kommen. Damit ist ein einfaches Verbinden dieser Enden miteinander über den Feststell- und Befestigungsmechanismus 24 möglich. Zur leichteren Betätigung des Feststell- und Befestigungsmechanismus 24 weist dieser ein hebelartiges Element auf.

Zudem wird aus Figur 3 deutlich, dass die Verbindungselemente 20, 22 jeweils als separate Elemente ausgebildet sind und im Fußgestell 14 befestigt sind. Dabei sind die entsprechenden Enden 16, 18 der entsprechenden Fußgestelle 14 der Geräteständer 10, 10' ungefähr U-förmig ausgebildet. Die Verbindungselemente 20, 22 sind innerhalb der von den jeweiligen Tragsäulen 12 wegweisenden Enden 16, 18 längenverstellbar befestigt. Es wird deutlich, dass in den genannten Enden 16, 18 der jeweiligen Fußgestelle 14 mehrere voneinander beabstandete Öffnungen 34 zur Aufnahme des Feststellbolzens 36 ausgebildet sind. Durch die Längenverstellbarkeit der Verbindungselemente 20, 22 ist es möglich die jeweiligen Strahlentherapievorrichtungen 44, 46 in vorbestimmten und definierten Abständen zueinander anzuordnen.

Figur 4 zeigt eine schematische Darstellung der Strahlentherapievorrichtung 44. In dem dargestellten Ausführungsbeispiel ist die Strahlentherapievorrichtung 44 für die UV-Ganzkörperbehandlung vorgesehen. Hierfür weist das Bestrahlungsgerät 28 entsprechende UV-Strahler 54 auf. Die Bestrahlungsstärke und/oder die Bestrahlungszeit kann dabei durch ein Steuergerät 56 geregelt werden. Die Spektralbereiche der Strahler 54 können dabei in Breitband UV-A, Breitband UV-B oder Schmalband UV-B liegen. Es ist aber möglich, dass neben den UV-Anwendungen auch reine Farblichtstrahler zum Einsatz kommen. Des Weiteren besteht die Möglichkeit, dass unterschiedliche Strahler 54 mit unterschiedlichen Spektralbereichen kombiniert werden. Des Weiteren erkennt man, dass die Längsachse des Bestrahlungsgerätes 28 vertikal ausgerichtet ist. Zudem weist das Bestrahlungsgerät 28 in der Draufsicht eine gekrümmte Form auf.

Das Bestrahlungsgerät 28 ist an dem Geräteständer 10 angeordnet, dadurch einerseits verfahrbar und andererseits mit weiteren Strahlentherapievorrichtungen ohne weiteres koppelbar. Die Kopplung bzw. Verbindung der einzelnen Strahlentherapievorrichtungen erfolgt wiederum über die Verbindungselemente 22, 24. In dem dargestellten Ausführungsbeispiel ist ein Strahlungsgerät 28 am Geräteständer 10 bzw. der Tragsäule 12 des Geräteständers 10 angeordnet. Es ist aber auch möglich mehrere Bestrahlungsgeräte übereinander an der Tragsäule 12 des Geräteständers 10 anzuordnen.

Figur 5 zeigt eine schematische Darstellung zweier miteinander verbundener Geräteständer 10, 10'. Die Ausgestaltung der Geräteständer 10, 10' entspricht dabei dem in Figur 2 beschriebenen Geräteständer 10 gemäß der zweiten Ausführungsform. Man erkennt, wie die beiden Geräteständer 10, 10' über die hakenförmigen Verbindungselemente 20, 22 miteinander verbunden werden. Zur Koppelung der Verbindungselemente 20, 22 wird wiederum der bereits beschriebene Feststell- und Befestigungsmechanismus 24 verwendet. Da die Geräteständer 10, 10' auf den Rollen 26 gelagert sind, sind sie leicht zueinander zu positionieren. Die Geräteständer 10, 10' können dabei z. B. in einer geraden Linie nebeneinander angeordnet werden. Es ist aber auch möglich, dass die Geräteständer halbkreisförmig oder kreisförmig zueinander aufgestellt und gekoppelt werden.

Figur 6 zeigt eine schematische Gesamtdarstellung des Strahlentherapiesystems 48 gemäß Figur 3. Man erkennt die Positionierung der beiden Strahlentherapievorrichtungen 44, 46 zueinander. Die Strahlentherapievorrichtungen 44, 46 umfassen dabei wiederum jeweils ein Bestrahlungsgerät 28, 28' deren Längsachse vertikal ausgerichtet ist. Durch die gekrümmte Ausgestaltung der Bestrahlungsgeräte 28, 28' ergibt sich durch die Koppelung über die Verbindungselemente 20, 22 der jeweiligen Geräteständer 10, 10' ein Raum vor den Bestrahlungsgeräten 28, 28', der für einen Benutzer des Strahlentherapiesystems 48 vorgesehen ist. Durch die Verbindungselemente 20, 22 wird dieser Raum und der Abstand der einzelnen Strahlentherapievorrichtungen 44, 46 eindeutig definiert. In dem dargestellten Ausführungsbeispiel bilden die beiden Strahlentherapievorrichtungen 44, 46 ungefähr einen Halbkreis aus, der einem Benutzer des Strahlentherapiesystems 48 zumindest teilweise umgibt. Man erkennt, dass das Strahlentherapiesystem 48 ortsveränderlich, fahrbar und flexibel ausgestaltbar ist. Es dient insbesondere zur Behandlung von dermatologischen und kosmetischen Hautkrankheiten und Hautveränderungen. Das Strahlentherapiesystem 48 kann dabei mit einer unterschiedlichen Anzahl an Strahlentherapievorrichtungen 44, 46 versehen sein. Durch verschieden einsetzbare Bestrahlungsvorrichtungen 28, 28' sind unterschiedliche dermatologische und kosmetische Behandlungen möglich. Das Strahlentherapiesystem 48 kann mit einer Zeit- und/oder Dosissteuerung ausgestattet sein. Zudem ist eine fest voreingestellte Bestrahlungsstärke oder eine aktive Messung der Bestrahlungsstärke mit Ermittlung der daraus resultierenden Dosis durch zum Beispiel UV-Sensoren möglich. Die beweglichen Geräteständer 10, 10' ermöglichen dabei eine variable Anordnung der einzelnen Strahlentherapievorrichtungen 44, 46 zueinander. Damit sind die Strahlentherapievorrichtungen 44, 46 sowohl als Einzelgeräte als auch als Strahlentherapiesystem 48 mit mehreren Strahlentherapievorrichtungen 44, 46 verwendbar. Das Strahlentherapiesystem 48 kann damit ohne weiteres an die Bedürfnisse der Anwender und die räumlichen Gegebenheiten angepasst werden. Bezüglich der Merkmale der Bestrahlungsgeräte 28, 28' verweisen wir auf die entsprechende Beschreibung dieser Geräte in Figur 4.

Das Strahlentherapiesystem 48 kann auch mindestens drei Strahlentherapievorrichtungen aufweisen, die wiederum über die Verbindungselemente der Geräteständer miteinander gekoppelt sind. Die Koppelung kann dabei derart erfolgen, dass die Strahlentherapievorrichtungen einen kabinenartigen Raum für den Aufenthalt eines Benutzers des Strahlentherapiesystems 48 umschließen. So können zum Beispiel vier Strahlentherapievorrichtungen einen geschlossenen oder fast geschlossenen kabinenartigen Raum ausbilden. Dabei kann eine der vier Strahlentherapievorrichtungen nicht mit der nächstliegenden Strahlentherapievorrichtung über die entsprechenden Verbindungselemente verbunden sein, so dass dadurch eine Art Tür entsteht, die notwendig ist, um den umschlossenen, kabinenartigen Raum betreten und verlassen zu können. Die modulartige Ausgestaltung des Strahlentherapiesystems 48 ermöglicht eine Vielzahl von Gerätevarianten, die sich je nach Anwendung und den räumlichen Gegebenheiten richten kann.

## Patentansprüche

1. Geräteständer für eine Strahlentherapievorrichtung, insbesondere für die UV-Bestrahlung und UV-Therapie, mit mindestens einer Tragsäule (12) umfassend mindestens einen Feststellmechanismus zur Befestigung von mindestens einem Bestrahlungsgerät (28, 28'), wobei die Tragsäule (12) an einem ungefähr U-, sichel- oder halbkreisförmigen, verfahrbaren Fußgestell (14) abgestützt ist,
**dadurch gekennzeichnet,**
**dass** das Fußgestell (14) mindestens im Bereich eines von der Tragsäule (14) wegweisenden Endes (16, 18) mindestens ein hakenförmiges Verbindungselement (20, 22) aufweist.

2. Geräteständer nach Anspruch 1,
**dadurch gekennzeichnet,**
**dass** das Verbindungselement (20, 22) als separates Element ausgebildet und am oder im Fußgestell (14) lösbar oder nicht-lösbar befestigt ist.

3. Geräteständer nach Anspruch 2,
**dadurch gekennzeichnet,**
**dass** das Fußgestell (14) mindestens im Bereich eines des von der Tragsäule (12) wegweisenden Endes (16, 18) hohlförmig zur Aufnahme des Verbindungselementes (20, 22) ausgebildet ist.

4. Geräteständer nach Anspruch 3,
**dadurch gekennzeichnet,**
**dass** das hakenförmige Verbindungselement (20, 22) derart in oder an dem Ende (16, 18) des Fußgestells (14) angeordnet ist, dass ein nicht innerhalb des Fußgestells (14) angeordnetes oder nicht mit dem Fußgestell (14) abschließendes Ende (30, 32) des Verbindungselements (20, 22) in einer von dem Fußgestell (14) wegzeigenden Richtung verläuft.

5. Geräteständer nach einem der vorhergehenden Ansprüche,
**dadurch gekennzeichnet,**
**dass** das Verbindungselement (20, 22) mindestens einen Feststell- und Befestigungsmechanismus (24) zur Verbindung eines Endes (30, 32) des Verbindungselements (20, 22) des Geräteständers (10) mit einem entsprechenden Ende (30, 32) eines Verbindungselements (20, 22) eines zweiten Geräteständers (10') aufweist.

6. Geräteständer nach einem der vorhergehenden Ansprüche,
**dadurch gekennzeichnet,**
**dass** das Verbindungselement (20, 22) innerhalb des von der Tragsäule (12) wegweisenden Endes (16, 18) längenverstellbar befestigt ist.

7. Geräteständer nach Anspruch 6,
**dadurch gekennzeichnet,**
**dass** das von der Tragsäule (12) wegweisende Ende (16, 18) des Fußgestells (14) mindestens zwei voneinander beabstandete Öffnungen (34) zur Aufnahme eines Feststellbolzens (36) aufweist, wobei der Feststellbolzen (36) in eine entsprechende Öffnung in dem Verbindungselement (20, 22) einbringbar ist.

8. Geräteständer nach einem der vorhergehenden Ansprüche,
**dadurch gekennzeichnet,**
**dass** die Tragsäule (12) und/oder das Fußgestell (14) einen quadratischen, rechteckigen oder kreisförmigen Querschnitt ausweisen.

9. Geräteständer nach einem der vorhergehenden Ansprüche,
**dadurch gekennzeichnet,**
**dass** die Tragsäule (12) höhenverstellbar ausgebildet ist.

10. Geräteständer nach einem der vorhergehenden Ansprüche,
**dadurch gekennzeichnet,**
**dass** das Fußgestell (14) einen Querträger (38) und zwei ungefähr senkrecht von den Enden des Querträgers (38) abzweigende Seitenarme (40, 42) umfasst.

11. Geräteständer nach einem der vorhergehenden Ansprüche,
**dadurch gekennzeichnet,**
**dass** der Feststellmechanismus zur lösbaren Befestigung des mindestens einen Bestrahlungsgerätes (28, 28') ein Schwenken des Bestrahlungsgerätes (28, 28') ermöglicht.

12. Strahlentherapievorrichtung, insbesondere für die UV-Bestrahlung und UV-Therapie, umfassend einen Geräteständer (10, 10') gemäß einem der Ansprüche 1 bis 11 und mindestens ein Bestrahlungsgerät (28, 28') welches an einem Geräteständer (10, 10') gemäß einem der Ansprüche 1 bis 11 befestigt ist.

13. Strahlentherapievorrichtung nach Anspruch 12,
**dadurch gekennzeichnet,**
**dass** das Bestrahlungsgerät (28, 28') in der Draufsicht gekrümmt oder gebogen ausgebildet ist.

14. Strahlentherapiesystem umfassend mindestens zwei über ihre Geräteständer (10, 10') lösbar miteinander verbundene Strahlentherapievorrichtungen (44, 46) nach Anspruch 12 oder 13.

15. Strahlentherapiesystem nach Anspruch 14,
**dadurch gekennzeichnet,**
**dass** mindestens drei Strahlentherapievorrichtungen miteinander gekoppelt sind, derart, dass sie einen kabinenartigen Raum für den Aufenthalt eines Benutzers des Strahlentherapiesystems (48) umschließen.

## Claims

1. Appliance stand for a radiotherapy device, in particular for UV irradiation and UV therapy, including at least one supporting column (12) comprising at least one locking mechanism for fixing at least one irradiation appliance (28, 28'), wherein the supporting column (12) is supported on an approximately U-shaped, crescent-shaped or semicircular, movable pedestal (14),
**characterized in that**
the pedestal (14) has at least one hook-shaped connecting element (20, 22) at least in the area of an end (16, 18) facing away from the supporting column (14).

2. Appliance stand according to claim 1,
**characterized in that**
the connecting element (20, 22) is formed as a separate element or is detachably or non-detachably fixed to or in the pedestal (14).

3. Appliance stand according to claim 2,
**characterized in that**
the pedestal (14) is formed hollow for receiving the connecting element (20, 22) at least in the area of the end (16, 18) facing away from the supporting column (12).

4. Appliance stand according to claim 3,
**characterized in that**
the hook-shaped connecting element (20, 22) is disposed in or at the end (16, 18) of the pedestal (14) such that an end (30, 32) of the connecting element (20, 22) not disposed within the pedestal (14) or not terminating with the pedestal (14) extends in a direction pointing away from the pedestal (14).

5. Appliance stand according to any one of the preceding claims,
**characterized in that**
the connecting element (20, 22) has at least one locking and fixing mechanism (24) for connecting an end (30, 32) of the connecting element (20, 22) of the appliance stand (10) to a corresponding end (30, 32) of a connecting element (20, 22) of a second appliance stand (10').

6. Appliance stand according to any one of the preceding claims,
**characterized in that**
the connecting element (20, 22) is fixed adjustable in length within the end (16, 18) facing away from the supporting column (12).

7. Appliance stand according to claim 6,
**characterized in that**
the end (16, 18) of the pedestal (14) facing away from the supporting column (12) has at least two openings (34) spaced from each other for receiving a locking bolt (36), wherein the locking bolt (36) is insertable into a corresponding opening in the connecting element (20, 22).

8. Appliance stand according to any one of the preceding claims,
**characterized in that**
the supporting column (12) and/or the pedestal (14) have a square, rectangular or circular cross-section.

9. Appliance stand according to any one of the preceding claims,
**characterized in that**
the supporting column (12) is formed adjustable in height.

10. Appliance stand according to any one of the preceding claims,
**characterized in that**
the pedestal (14) includes a crossbeam (38) and two lateral arms (40, 42) branching approximately perpendicularly from the ends of the crossbeam (38).

11. Appliance stand according to any one of the preceding claims,
**characterized in that**
the locking mechanism for detachably fixing the at least one irradiation appliance (28, 28') allows pivoting of the irradiation appliance (28, 28').

12. Radiotherapy device, in particular for UV irradiation and UV therapy, including an appliance stand (10, 10') according to any one of claims 1 to 11, and at least one irradiation appliance (28, 28'), which is fixed to an appliance stand (10, 10') according to any one of claims 1 to 11.

13. Radiotherapy device according to claim 12,
**characterized in that**
the irradiation appliance (28, 28') is formed curved or bent in plan view.

14. Radiotherapy system including at least two radiotherapy devices (44, 46) according to claim 12 or 13 detachably connected to each other via their appliance stands (10, 10').

15. Radiotherapy system according to claim 14,
**characterized in that**
at least three radiotherapy devices are coupled to each other such that they enclose a cabin-like space for the stay of a user of the radiotherapy system (48).

## Revendications

1. Support d'appareil pour un dispositif de radiothérapie, en particulier pour le rayonnement UV et la thérapie UV, avec au moins une potence (12), comprenant au moins un mécanisme de blocage destiné à fixer au moins un appareil de rayonnement (28, 28'), moyennant quoi la potence (12) est appuyée contre un piètement (14) à peu près en U, en forme de croissant ou semi-circulaire, déplaçable,
**caractérisé en ce**
**que** le piètement (14) présente, au moins dans le secteur d'une extrémité (16, 18) qui s'écarte du piètement (14), au moins un élément de raccordement (20, 22) en forme de crochet.

2. Support d'appareil selon la revendication 1,
**caractérisé en ce**
**que** l'élément de raccordement (20, 22) est constitué comme élément séparé et qu'il est fixé de manière amovible ou non amovible sur ou dans le piètement (14).

3. Support d'appareil selon la revendication 2,
**caractérisé en ce**
**que** le piètement (14) est constitué, au moins dans le secteur d'une extrémité (16, 18) qui s'écarte de la potence (12), en creux par rapport au logement de l'élément de raccordement (20, 22).

4. Support d'appareil selon la revendication 3,
**caractérisé en ce**
**que** l'élément de raccordement (20, 22) en forme de crochet est agencé dans ou sur l'extrémité (16, 18) du piètement (14) de telle sorte qu'une extrémité (30, 32) de l'élément de raccordement (20, 22), non agencée à l'intérieur du piètement (14) ou ne se terminant pas avec le piètement (14), évolue dans une direction qui s'écarte du piètement (14).

5. Support d'appareil selon l'une des revendications précédentes,
**caractérisé en ce**
**que** l'élément de raccordement (20, 22) présente au moins un mécanisme de blocage et de fixation (24) destiné à raccorder une extrémité (30, 32) de l'élément de raccordement (20, 22) du support d'appareil (10) à une extrémité (30, 32) correspondante d'un élément de raccordement (20, 22) d'un second support d'appareil (10').

6. Support d'appareil selon l'une des revendications précédentes,
**caractérisé en ce**
**que** l'élément de raccordement (20, 22) est fixé, en étant réglable en longueur, à l'intérieur de l'extrémité (16, 18) qui s'écarte de la potence (12).

7. Support d'appareil selon la revendication 6,
**caractérisé en ce**
**que** l'extrémité (16, 18) du piètement (14), qui s'écarte de la potence (12), présente au moins deux ouvertures (34), espacées l'une de l'autre, destinées à loger un boulon de blocage (36), moyennant quoi le boulon de blocage (36) peut être introduit dans une ouverture correspondante dans l'élément de raccordement (20, 22).

8. Support d'appareil selon l'une des revendications précédentes,
**caractérisé en ce**
**que** la potence (12) et / ou le piètement (14) présentent une section quadratique, rectangulaire ou circulaire.

9. Support d'appareil selon l'une des revendications précédentes,
**caractérisé en ce**
**que** la potence (12) est constituée en étant réglable en hauteur.

10. Support d'appareil selon l'une des revendications précédentes,
**caractérisé en ce**
**que** le piètement (14) comprend une barre transversale (38) et deux bras latéraux (40, 42) qui se détachent, à peu près verticalement, des extrémités de la barre transversale (38).

11. Support d'appareil selon l'une des revendications précédentes,
**caractérisé en ce**
**que** le mécanisme de blocage, destiné à fixer de manière amovible le au moins un appareil de rayonnement (28, 28'), permet un pivotement de l'appareil de rayonnement (28, 28').

12. Dispositif de radiothérapie, en particulier pour le rayonnement UV et la thérapie UV, comprenant un support d'appareil (10, 10') conformément à l'une des revendications 1 à 11 et au moins un appareil de rayonnement (28, 28'), qui est fixé à un support d'appareil (10, 10') conformément à l'une des revendications 1 à 11.

13. Dispositif de radiothérapie selon la revendication 12,
**caractérisé en ce**
**que** l'appareil de rayonnement (28, 28') est constitué curviligne ou courbé, vu de dessus.

14. Système de radiothérapie, comprenant au moins deux dispositifs de radiothérapie (44, 46), reliés ensemble de manière amovible par l'intermédiaire de leurs supports d'appareil (10, 10'), selon la revendication 12 ou 13.

15. Système de radiothérapie selon la revendication 14,
**caractérisé en ce**
**qu'**au moins trois dispositifs de radiothérapie sont couplés ensemble, de telle sorte qu'ils entourent un espace de type cabine pour le séjour d'un utilisateur du système de radiothérapie (48).
